(19) Europäisches Patentamt
European Patent Office
Office européen des brevets

(11) Publication number: 0 210 761
A1

(12) EUROPEAN PATENT APPLICATION

(21) Application number: 86305125.6

(22) Date of filing: 02.07.86

(51) Int. Cl.4: C 12 N 9/02
A 61 K 37/50

(30) Priority: 05.07.85 JP 148922/85

(43) Date of publication of application: 04.02.87 Bulletin 87/6

(84) Designated Contracting States: AT BE CH DE FR GB IT LI LU NL SE

(71) Applicant: Takeda Chemical Industries, Ltd.
27, Doshomachi 2-chome Higashi-ku
Osaka-shi Osaka, 541(JP)

(72) Inventor: Miyata, Kouichi
6-77, Seiwadainishi 1-chome
Kawanishi Hyogo 666-01(JP)

(72) Inventor: Nakagawa, Yasushi
1-7, Midoridai 5-chome
Kawanishi Hyogo 666-01(JP)

(72) Inventor: Nakamura, Masahira
408, Kitaimaichi Kashiba-cho
Kitakatsuragi-gun Nara 639-02(JP)

(74) Representative: Lewin, John Harvey et al,
Elkington and Fife High Holborn House 52/54 High Holborn
London WC1V 6SH(GB)

(54) **Modified enzymes, production and use thereof.**

(57) The present invention provides a modified enzyme of the formula:

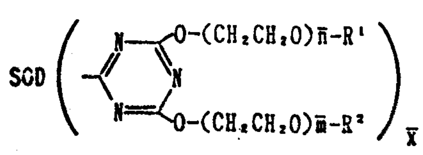

wherein SOD is a manganese-containing superoxide dismutase produced by a microorganism of the genus *Serratia*; n and m are the same or different and each is equal to about 7 to 700; $R^1$ and $R^2$ are the same or different and each is a hydroxy-protecting group; and x is equal to about 1 to 30.

The modified enzyme of the present invention shows increased antiinflammatory activity, is substantially free from immunogenicity and shows a prolonged half-life time and higher level in blood, hence it can advantageously be used as an antiinflammatory agent.

Modified Enzymes, Production and Use Thereof

The present invention relates to a modified enzyme of a manganese-containing superoxide dismutase (hereinafter referred to briefly as SOD) produced by a microorganism of the genus Serratia, which can be used as, for example, an antiinflammatory agent, a method for producing and a use of said modified enzyme.

The manganese-containing superoxide dismutase produced by a microorganism of the genus Serratia is known from EP Publication (laid open) No. 45222 and No. 70656.

Modification of asparaginase with polyethylene glycol has been reported [Chemistry Letters, 773-776 (1980)].

SOD as it is has an antiinflammatory activity but has the disadvantage of a short half life time in the blood circulation. Moreover, as SOD is an enzyme of microbial origin, it may present the problem of immunogenicity of the animals administered, due to foreign protein.

To overcome the above problems, various chemical modifications of SOD were attempted, and it was found that introduction of polyethylene glycol (hereinafter sometimes referred to briefly as PEG) to SOD results in very useful characteristics. While several techniques are available for modifying a protein with polyethylene

glycol, it has been found that the method in which activated two chains of polyethylene glycol with triazine covalently binds to the N-terminal amino acid or the ε-amino group of the lysine residue of SOD. This method is most advantageous in that the amounts of reaction by-products are small and the reaction yield is high. This finding and subsequent research led to the establishment of the present invention.

The present invention is directed to a modified enzyme of the formula:

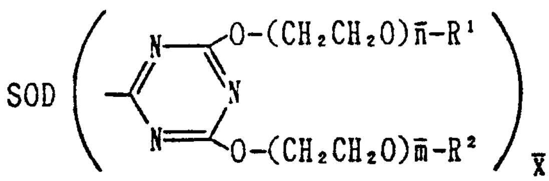

[I]

wherein SOD is a manganese-containing superoxide dismutase produced by a microorganism of the genus Serratia; $\bar{n}$ and $\bar{m}$ are the same or different and each is equal to about 7 to 700; $R^1$ and $R^2$ are the same or different and each is a hydroxy-protecting group; and $\bar{x}$ is equal to about 1 to 30, a method for producing a modified enzyme of formula [I] characterized by modifying SOD with polyethylene glycol and a use of the modified enzyme [I] for treatment of inflammation in mammals.

The manganese-containing superoxide dismutase produced by a microorganism of the genus Serratia may for example be the one obtained by the method described in EP Publication (laid open) No. 45222 or No. 70656.

In the formula given above, $\bar{n}$, $\bar{m}$ and $\bar{x}$ each represents a mean value; $\bar{n}$ and $\bar{m}$ may be the same or different, preferably $\bar{n}$ and $\bar{m}$ are the same and each is equal to about 7 to 700 and, preferably, about 7 to 250, more preferably about 30 to 150; and $\bar{x}$ is equal

to about 1 to 30 and, preferably, about 5 to 15.

The polyethylene glycol used in accordance with the present invention should preferably have an average molecular weight of about 300 to about 30,000, more preferably about 1,000 to about 10,000.

As examples of the hydroxy-protecting groups represented by $R^1$ and $R^2$, there may be mentioned alkyl groups containing 1-3 carbon atoms (e.g. methyl, ethyl, n-propyl, isopropyl), preferably methyl.

The modification of SOD with PEG may preferably be carried out via a triazine residue.

Such modification can be carried out by reaction of a mixture of a PEG of the general formula

$$HO-(CH_2CH_2O)_{\bar{n}}-R^1 \qquad [II]$$

wherein $\bar{n}$ and $R^1$ are as defined above, and a PEG of the general formula

$$HO-(CH_2CH_2O)_{\bar{m}}-R^2 \qquad [II']$$

wherein $\bar{m}$ and $R^2$ are as defined above, with a compound of the formula

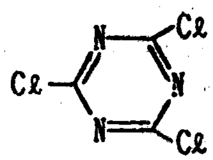

[III]

and then reacting the resulting compound of the general formula

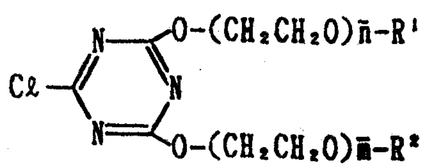

[IV]

wherein $\bar{n}$, $\bar{m}$, $R^1$ and $R^2$ are as defined above, with SOD.

For producing the compound [IV], the reaction of

the mixture of compound [II] and compound [II'] with the compound [III] is preferably carried out under anhydrous conditions. Thus, the reaction is carried out in benzene or toluene at about 50° to about 80°C for about 15 to 50 hours.

The mixture of compound [II] and compound [II'] is preferably used in an amount such that the total number of moles of the compounds [II] and [II'] is twice the number of moles of the compound [III]. After the reaction, aliphatic hydrocarbon-organic solvent, such as petroleum ether, is added to the reaction mixture to thereby cause precipitation of the compound [IV]. In some instances, gel filtration using an organic solvent (e.g. chloroform, acetonitrile, benzene, toluene), for instance, may be employed combinedly.

For the production of the compound [I] by reaction of the compound [IV] with SOD, the reaction is carried out in an aqueous medium for about 2 to 24 hours under mild conditions to avoid enzyme inactivation, for example at room temperature or below and at a pH of about 8 to about 10. After reaction, the reaction mixture is subjected to gel filtration and ultrafiltration in an aqueous solution to separate the compound [I] and the remaining compound [IV].

By these procedures, the compound [I], a PEG-modified SOD, is obtained, hence hereinafter sometimes referred to as "PEG-SOD".

The compound [I] according to the invention has sufficiently high SOD activity. Testing of the compound for evaluating antiinflammatory agents [I] by such model experiments as the carrageenin abscess model, delayed type IV allergic reaction with ovalbumin, Masugi nephritis model and radiation damage has revealed that the PEG-modified SOD is generally superior in pharmacological effects to the original SOD.

As compared with the original SOD, the compound [I] has the following advantageous features:

(1) It increases antiinflammatory activity.

(2) It decreases immunogenicity.

(3) Its half-life time in blood circulation is longer.

(4) One time injection of it induces immunological tolerance.

(5) Blood level of SOD is much higher after intraduodenal administration.

Furthermore, the compound [I] has low toxicity.

In this manner, the compound [I] has excellent activity and properties and, in addition, is low in toxicity. Therefore, it can be used advantageously as an antiinflammatory agent.

In using the compound [I] according to the invention as an antiinflammatory agent, it is administered either orally or parenterally for the purpose of treating various acute or subacute inflammations [e.g. edema, (e.g. arthritis, bronchitis, bruise-caused edema, burn, radiation)] in mammals (e.g. mouse, rat, rabbit, cat, dog, monkey, human), for instance.

For administration, the compound [I] is mixed with a pharmacologically acceptable carrier, excipient, diluent, etc., and made up into preparations for oral administration, such as tablets or capsules, or into preparations for parenteral administration, such as injections, and is administered to the above mammals in such preparation form by the <u>per se</u> known method.

The daily dose of the compound [I] is such that the enzyme quantity in the compound [I] amounts to about 0.1 to about 100 mg, preferably about 0.5 to 50 mg, per kilogram of body weight.

<u>Brief Description of the Drawings</u>

Fig. 1 shows the results of immunological tolerance induction as obtained in Test Example 6.

Fig. 2 shows the results of plasma level of SOD activity after intraduodenal administration to rats as disclosed in Test Example 7.

Examples

The following working examples, reference example and test examples are further illustrative of the present invention.

Example 1 Production of PEG-SOD

Cyanuric chloride (730 mg) was added to a mixture of 40 g of monomethoxy polyethylene glycol (average molecular weight: 5,000), 200 ml of benzene, 20 g of anhydrous sodium carbonate and 10 g of molecular sieve 3A (Wako Pure Chemical Industries, Japan), and the reaction was carried out by stirring at 80°C for 20 hours. Thereafter, 400 ml of petroleum ether was added to thereby cause precipitation of the activated PEG. The precipitate was dissolved in benzene, followed by the precipitation by the same procedure. After three-time repetitions of this precipitation procedure, the precipitate (activated PEG) was dried in a desiccator under reduced pressure.

A 1.5 g portion of the above activated PEG was added to 15 mg of an SOD prepared from Serratia marcescens ATCC 21074 by the procedure described in EP Publication (laid open) No. 70656 (The mole ratio of the activated PEG to the SOD was 300.) The reaction was carried out at 4°C for 2 hours in 5.0 ml of 0.1 M borate buffer (pH 9.0) and then terminated by adding 30 ml of 0.1 M phosphate buffer (pH 7.0). The residual PEG was removed by ultrafiltration [Amicon (USA) membrane YM-10]. The concentrate (2.0 ml) of PEG-SOD was purified by gel filtration using a Sephacryl S-200 (Pharmacia, Sweden) column (5 x 73 cm). The thus-obtained PEG-SOD, weighed about 15 mg, was modified 7-12 amino residues of the original SOD, and its SOD

activity retained about 50%. The ultraviolet absorption at 280 nm was increased by less than 5% of the original. At shorter wavelengths, the absorptions due to the triazine residue were observed.

Example 2 Production of PEG-SOD

PEG-SODs were prepared by the same method as in Example 1 using monomethoxy polyethylene glycols differing in molecular weight (average molecular weight: 350, 750 and 1,900, respectively) with the SOD used in Example 1. The results thus obtained are shown in Table 1.

Table 1

| | Specific activity (units/mg protein) | Amino group modification percentage |
|---|---|---|
| SOD (original) | 100% | 0% |
| PEG(350)-SOD | 39% | 48% |
| PEG(750)-SOD | 41% | 48% |
| PEG(1,900)-SOD | 41% | 48% |

Note: The value in the parentheses after "PEG" indicates the average molecular weight of the PEG used.

Reference Example 1 Preparation of dextran-SOD

In 50 ml of distilled water was dissolved 1 g of dextran (average molecular weight: 60,000-90,000), followed by addition of about 0.3 g of cyanogen bromide. The reaction was carried out at 20°C for 30 minutes while the pH was maintained at 10.5 by addition of 5 N NaOH. Then, the pH was adjusted to 9.0 with $NaHCO_3$, the SOD used in Example 1 (114 mg in 13 ml of distilled water) was added, and the reaction was carried out furthermore at 4°C for 20 hours. The reaction mixture was concentrated to 3 ml by ultrafiltration (using YM-10) and the remaining SOD was removed by gel

filtration using a Sephacryl S-200 column (5 x 73 cm). Thus was obtained about 0.7 g of dextran-SOD. The dextran-SOD modified with 34% of the amino groups had a residual enzymatic activity of 67% of the original SOD.

Test Example 1 Antiinflammatory activity

The carrageenin abscess test was used as an evaluation method of antiinflammatory activity. Thus, as described in Acta Pharmacologica Japonica, 84, 337 (1984), a 2% (w/v) carrageenin solution was subcutaneously injected into the dorsal region of rats. Thirty minutes later, the enzymes show in Table 2 were each administered intravenously. After further 24 hours, each abscess was excised and weighed for comparison with the control group (given physiological saline).

In this and the subsequent test examples, the SOD used was same used in Example 1 and the dextran-SOD used was the product obtained in Reference Example 1 described above.

Table 2 Antiinflammatory activity (carrageenin abscess method)

| | Dose 1) | Number of animal | Abscess weight 2) | Inhibition |
|---|---|---|---|---|
| Control | - | 10 | 2.117 ± 0.11 g | - |
| SOD | 2 mg/kg | 10 | 2.065 ± 0.13 g | 2.5% |
| Dextran-SOD | 2 mg/kg | 10 | 1.695 ± 0.12 g 3) | 20.5% |
| PEG-SOD 4) | 2 mg/kg | 10 | 1.701 ± 0.11 g 3) | 19.7% |

Notes: 1) Expressed in terms of the number, the doses were same as SOD activity.

2) Mean ± standard error

3) $P < 0.05$ (Student's T test)

4) The PEG-SOD obtained in Example 1 was used.

As shown in Table 2, the modified enzymes, dextran-SOD and PEG-SOD, increased antiinflammatory activity as compared with the original SOD and the efficacy of the modified enzymes showed statistically significant as compared with that of the control group (given physiological saline).

Test Example 2 Immunogenicity

The method described in Journal of Immunological Methods, 14, 381 (1977) was used. Each enzyme (20 μg as SOD) was emulsified with Freund's complete adjuvant (FAC) and the emulsion (0.2 ml) was administered intraperitoneally to A/J mice, followed by additional administration on days 14 and 28. Blood samples were taken from the mouse orbital vein every 7 day interval after the day of the first administration. The sera from the blood samples were evaluated for antibody production by the passive cutaneous anaphylaxis (PCA) reaction method. Rats were intradermally injected with a diluted serum (0.1 ml), followed by injecting 2 ml of a solution containing 0.5 mg of SOD and 20 mg of Evans blue 4 hours later and the vascular permeability visualized by the dye was determined. The results thus obtained are shown in Table 3. The values in the table indicate the maximum serum dilution factor capable of giving positive PCA reaction.

Table 3 Immunogenicity testing

| | Number of animal | PCA titer judged on Day 7 | Day 14 | Day 21 | Day 28 | Day 35 |
|---|---|---|---|---|---|---|
| SOD | 8 | 8 | 32 | 256 | 256 | 256 |
| Dextran-SOD | 8 | 0 | 256 | 128 | 128 | 256 |
| PEG-SOD 1) | 8 | 0 | 0 | 0 | 0 | 0 |

Note 1) The PEG-SOD used was the product obtained in Example 1.

As shown in Table 3, the PEG-SOD showed no antibody production while the original SOD and dextran-SOD caused antibody production against SOD. In a test conducted by the similar method, no antibody production against PEG-SOD was observed in the PEG-SOD administered rats.

Test Example 3 Half-life time in blood circulation

Each enzyme (5 mg/kg as SOD) was intravenously injected (1 ml) into rats (3 per group). Blood samples were taken from the caudal vein at time intervals and measured for serum SOD activity, and the half-life time was determined based on the first order reaction rate constant.

Table 4 Half-life time in blood circulation

| | |
|---|---|
| SOD | 1.4 hours |
| PEG-SOD[1)] | 14.2 hours |

Note 1) The PEG-SOD obtained in Example 1 was used.

As shown in Table 4, the half-life time of PEG-SOD in the blood circulation was about 10 times longer as compared with that of the original SOD.

Test Example 4 Half-life time in blood circulation

The half-life time of SOD and the PEG-SODs obtained in Examples 1 and 2 were determined by the same described in Test Example 3. The results are shown in Table 5.

Table 5 Half-life time in blood circulation

| | Half-life time[1)] |
|---|---|
| SOD | 1.4 hours |
| PEG(350)-SOD[2)] | 6.3 hours |
| PEG(750)-SOD[2)] | 8.7 hours |
| PEG(1,900)-SOD[2)] | 11.2 hours |

Notes: 1) Determined by the same method described in Test Example 3.

2) The PEG-SODs obtained in Example 2 were used.

As shown in Table 5, the SOD's modified with various PEG's showed a prolonged half-life time, compared with that of the original SOD.

Test Example 5 Immunogenicity

The PEG-SODs obtained in Example 2 were tested for immunogenicity by the same method described in Test Example 2. The results are shown in Table 6.

Table 6 Immunogenicity test

| | Number of animal | PCA titer judged on Day 7 | Day 14 | Day 21 | Day 28 | Day 35 |
|---|---|---|---|---|---|---|
| SOD | 8 | 4 | 128 | 128 | 512 | 512 |
| PEG(350)-SOD[1)] | 8 | 0 | 8 | 32 | 256 | 256 |
| PEG(750)-SOD[1)] | 8 | 0 | 4 | 16 | 32 | 32 |
| PEG(1,900)-SOD[1)] | 8 | 0 | 0 | 0 | 0 | 0 |

Note 1) The PEG-SODs obtained in Example 2 were used.

As is evident from Table 6, the modification of SOD with PEG reduced antibody production. Especially for the PEG(1,900)-SOD, no antibody production was noted.

Test Example 6 Induction of immunological tolerance

A/J mice (8 per group) were intravenously injected with the PEG-SOD obtained in Example 1 at a dose of 20 μg SOD/mouse. An emulsion of 20 μg of SOD with Freund's complete adjuvant (FCA)/mouse was intraperitoneally administered 1, 3 and 5 weeks later. The titers of anti-SOD IgE antibody were determined by the PCA reaction described in Test Example 2. The results are shown in Fig. 1. As is apparent from Fig. 1, the initial administration of the PEG-SOD (shown by ---o---) completely suppressed the IgE antibody production against SOD even after the subsequent administrations of SOD. (Note: IgE = Immunoglobulin E.)

For the control, 20 μg of SOD/mouse was intravenously injected into A/J mice (8 per group) and an emulsion of 20 μg of SOD in FCA was administered intraperitoneally 1, 3 and 5 weeks later. The results are also shown in Fig. 1 by ——Δ——. As is apparent from Fig. 1, the initial administration of the original SOD elicited the IgE antibody production against SOD. The data indicated by ——●—— in Fig. 1 show the pattern of IgE antibody production against SOD when physiological saline was intravenously injected into A/J mice (8 per group) followed by intraperitoneal administration of an emulsion of 20 μg of SOD in FCA 1, 3 and 5 weeks later.

As in evident from the above results, the PEG-SOD induced immunological tolerance more strongly than the original SOD.

Test Example 7 Plasma level of SOD activity after intraduodenal administration

PEG-SOD obtained in Example 1 and SOD were administered intraduodenally through a cannula to rats (male, Jcl:Sprague-Dawley, 7 week old) at a dose of 100 mg as SOD/kg. Blood was taken from the rat tail vein 0,

1, 2, 4, 6, 10 and 24 hours after the administration and SOD activity in the plasma was assayed. The results are shown in Fig. 2. The plasma SOD level of the PEG-SOD administered rats presented by ——o—— was much higher than that of the SOD administered rats presented by ---Δ--- throughout the time course. Each point represents the mean with standard error (SE) of 5 rats in PEG-SOD and 3 rats in SOD.

What is claimed is:

1. A modified enzyme of the formula:

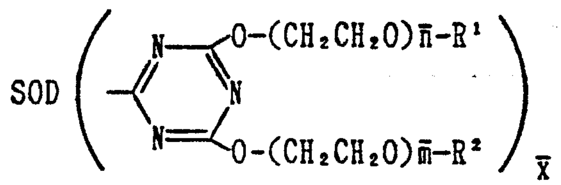

wherein SOD is a manganese-containing superoxide dismutase produced by a microorganism of the genus Serratia; $\bar{n}$ and $\bar{m}$ are the same or different and each is equal to about 7 to 700; $R^1$ and $R^2$ are the same or different and each is a hydroxy-protecting group; and $\bar{x}$ is equal to about 1 to 30.

2. The enzyme according to Claim 1, wherein $\bar{n}$ and $\bar{m}$ are the same.

3. The enzyme according to Claim 1, wherein $\bar{n}$ and $\bar{m}$ each is equal to about 7 to 250.

4. The enzyme according to Claim 1, wherein $R^1$ and $R^2$ are the same.

5. The enzyme according to Claim 1, wherein $R^1$ and $R^2$ each is $C_{1-3}$ alkyl.

6. The enzyme according to Claim 1, wherein SOD is produced by Serratia marcescens ATCC 21074.

7. A method of producing the enzyme according to Claim 1, which comprises reacting SOD with the compound of the formula:

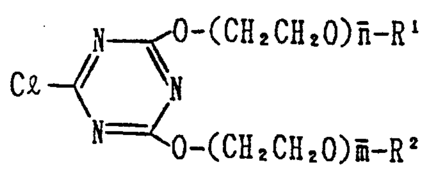

wherein $\bar{n}$, $\bar{m}$, $R^1$ and $R^2$ are defined in Claim 1.

8. A pharmaceutical composition which contains the enzyme according to Claim 1 and a pharmacologically acceptable carrier, excipient or diluent therefor.

9. The composition according to Claim 8, which is formulated to a tablet or capsule for oral administration.

10. A method for treatment of inflammation in a mammal, which comprises administering an effective amount of the enzyme according to Claim 1 to the mammal.

Fig. 1

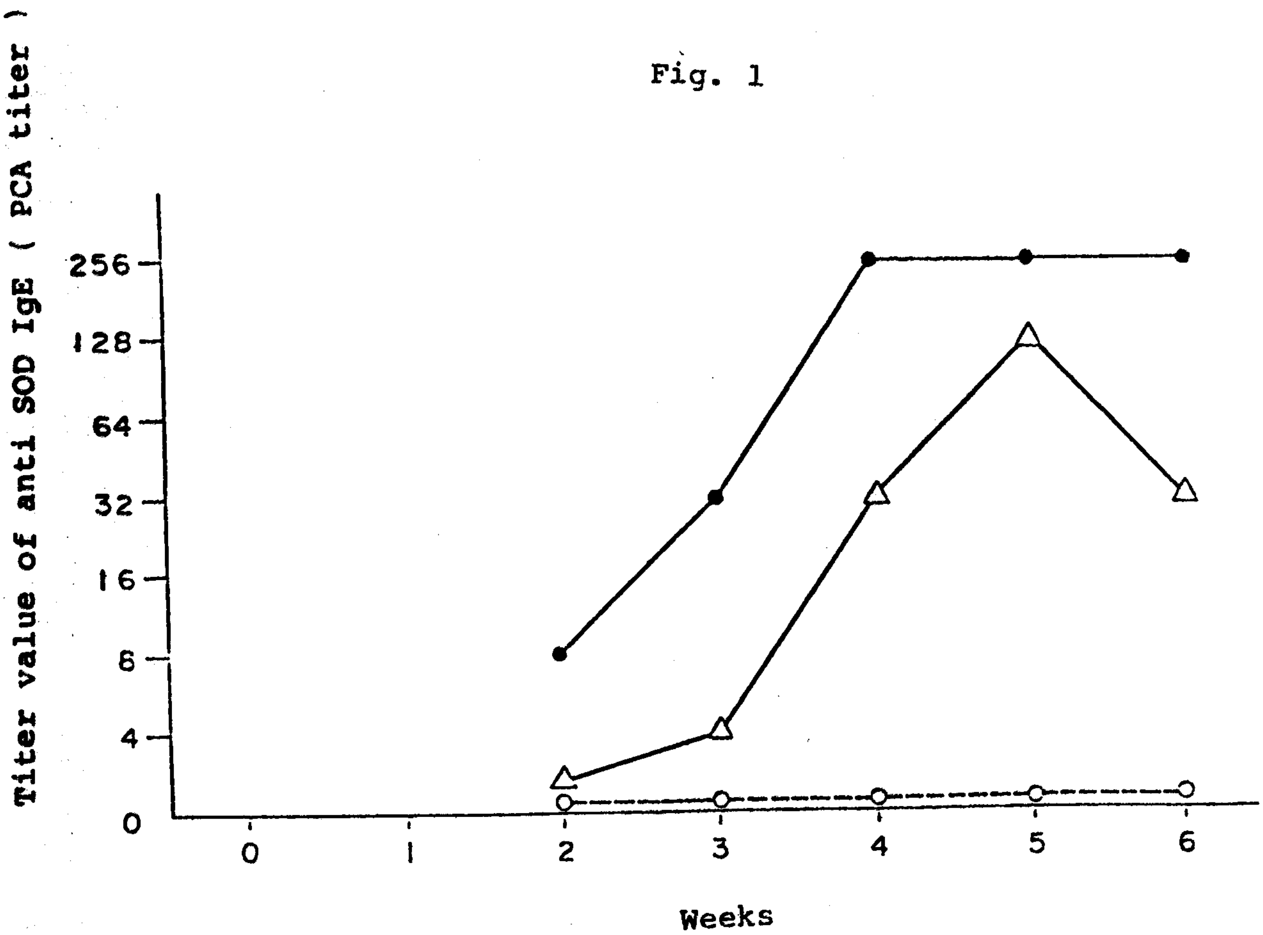

Titer value of anti SOD IgE ( PCA titer )
256
128
64
32
16
8
4
0
0
1
2
3
4
5
6
Weeks

Fig. 2

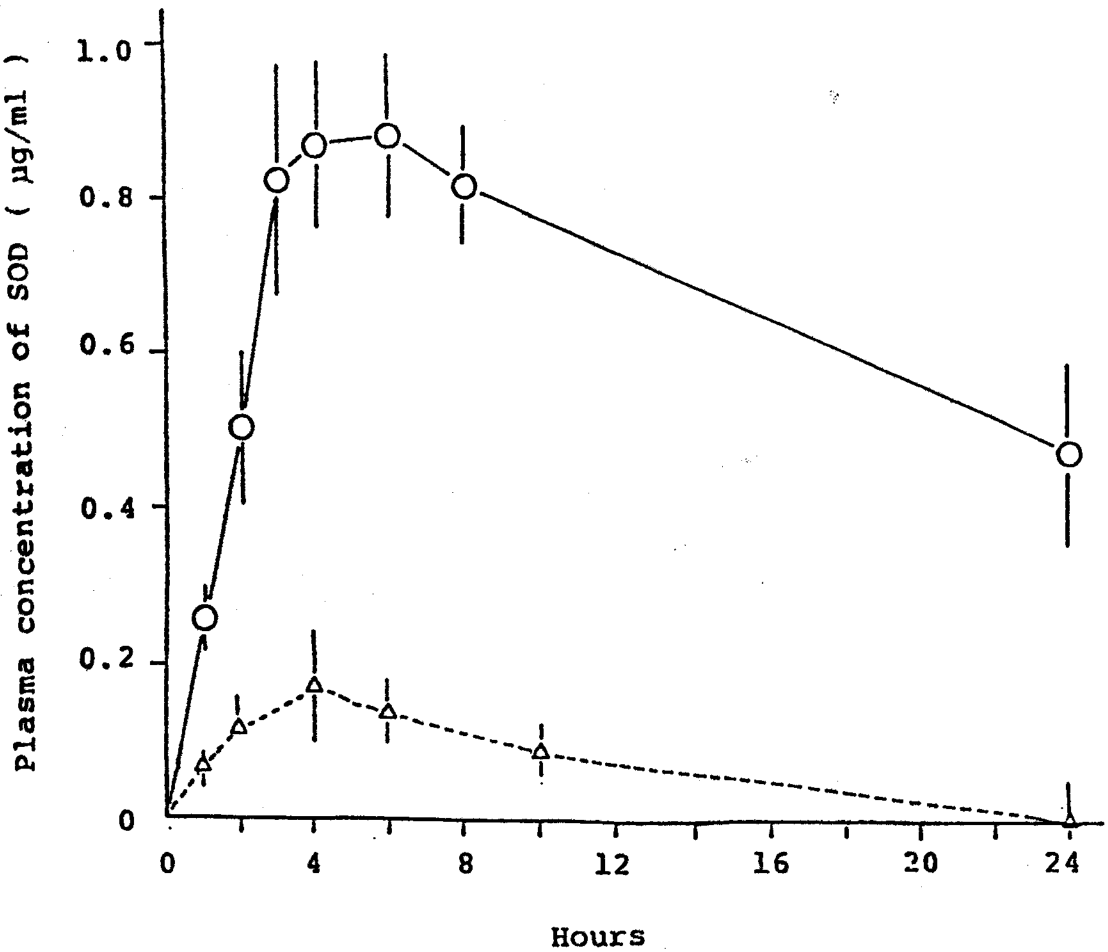
Plasma concentration of SOD ( µg/ml )
1.0
0.8
0.6
0.4
0.2
0
0
4
8
12
16
20
24
Hours

European Patent Office

Application number
EP 86 30 5125

# PARTIAL EUROPEAN SEARCH REPORT

which under Rule 45 of the European Patent Convention shall be considered, for the purposes of subsequent proceedings, as the European search report

## DOCUMENTS CONSIDERED TO BE RELEVANT

| Category | Citation of document with indication, where appropriate, of relevant passages | Relevant to claim | CLASSIFICATION OF THE APPLICATION (Int Cl 4) |
|---|---|---|---|
| X | RADIATION RESEARCH, volume 93, 1983, pages 581-587 B.H. GRAY: "Radioprotection by polyethylene glycol-protein complexes in mice" * page 582 * -- | 1-7 | C 12 N 9/02<br>A 61 K 37/50 |
| X | CHEMICAL ABSTRACTS, volume 93, no. 15, October 13, 1980 page 64, abstract 143000f COLUMBUS, OHIO (US) P.S. PYATAK et al.: "Preparation of a polyethylene glycol: superoxide dismutase adduct, and an examination of its blood circulating life and anti-inflammatory activity" & Res.Commun.Chem.Pathol. Pharmacol. 1980, 29(1), 113-27 * abstract * -- | 1-9 | |

TECHNICAL FIELDS SEARCHED (Int. Cl.4)

C 12 N 9
A 61 K 37

## INCOMPLETE SEARCH

./.

The Search Division considers that the present European patent application does not comply with the provisions of the European Patent Convention to such an extent that it is not possible to carry out a meaningful search into the state of the art on the basis of some of the claims.

Claims searched completely: 1-9

Claims searched incompletely:

Claims not searched: 10

Reason for the limitation of the search:

Method for treatment of the human or animal body by surgery or therapy (see art. 52 (4) of the European Patent Convention).

| Place of search | Date of completion of the search | Examiner |
|---|---|---|
| THE HAGUE | 25-09-1986 | SKELLY |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

0210761

European Patent Office

**PARTIAL EUROPEAN SEARCH REPORT**

Application number EP 86 30 5125 -2-

| DOCUMENTS CONSIDERED TO BE RELEVANT | | | CLASSIFICATION OF THE APPLICATION (Int. Cl.4) |
|---|---|---|---|
| Category | Citation of document with indication, where appropriate, of relevant passages | Relevant to claim | |
| X | CHEMICAL ABSTRACTS, volume 94, no. 7, February 16, 1981 page 226, abstract 43310j COLUMBUS, OHIO (US) F.F. DAVIS et al.: "Soluble, nonantigenic polyethylene glycol-bound enzymes" & Polym.Prepr.Am.Chem.Soc.Div. Polym. Chem.1979,20(1)357-60 (Eng). * abstract * | 1-9 | |
| Y | CHEMISTRY LETTERS, no. 7, July 1980, pages 773-776 The Chemical Soc. of Japan (JP) A. MATSUSHIMA et al.: "Modification of E.Coli asparaginase with 2,4-bis(O-methoxypolyethylene glycol)-6-chloro-S-triazine (activated $Peg_2$); disappearance of binding ability towards antiserum and retention of enzymic activity" * whole document * | 7 | TECHNICAL FIELDS SEARCHED (Int Cl 4) |
| Y,D | EP - A - 0 045 222 (TAKEDA CHEMICAL INDUSTRIES) * whole document * | 7 | |
| A,D | EP - A - 0 070 656 (TAKEDA CHEMICAL INDUSTRIES) * whole document * | 1-9 | |
| A | LA RECHERCHE, volume 10, no. 106 December 1979, page 1269 PARIS (FR) M. MICHELSON: "Une enzyme qui nous veut du bien" * whole document * | 1-9 | |

EPO Form 1505.3 06.78